# EUROPEAN PATENT SPECIFICATION

(11) **EP 0 487 719 B1**
(45) Date of publication and mention of the grant of the patent: **12.08.1998**
(21) Application number: 91913406.4
(22) Date of filing: 26.06.1991
(51) Int. Cl.: C07H 17/00

(54) **PROCESS FOR THE PREPARATION OF 9-BETA-D-ARABINOFURANOSYL-2-FLUOROADENINE 5'-PHOSPHATE**
VERFAHREN ZUR HERSTELLUNG VON 9-BETA-D-ARABINOFURANOSYL-2-FLUOROADENIN 5'-PHOSPHATEN
PROCEDE DE PREPARATION DE 9-BETA-ARABINOFURANOSYL-2-FLUOROADENINE 5'-PHOSPHATE

(30) Priority: 27.06.1990 US 544746
(43) Date of publication of application: 03.06.1992
(73) Proprietor: ASH STEVENS, INC., Detroit, Michigan 48202-3398 (US)
(72) Inventor: BLUMBERGS, Peter, Royal Oak, MI 48072 (US); KHAN, Mohammed, S., Azamgarh City, U.P.-276001 (IN); KALAMAS, Richard, L., Wyandotte, MI 48192 (US); PATEL, Ambalal, Troy, MI 48089 (US); LAMONTAGNE, Maurice, P., Farmington Hills, MI 48018 (US)
(74) Representative: Grünecker, Kinkeldey, Stockmair & Schwanhäusser Anwaltssozietät
(86) International application number: US9104566
(87) International publication number: WO9200312

(56) References cited:
- US-A- 3 413 282
- US-A- 4 093 714
- US-A- 4 123 609
- US-A- 4 136 175
- US-A- 4 357 324

## Description

This application is a continuation-in-part of U.S. Application Serial No. 445,446, filed December 4, 1989.

The present invention relates to an improved process for the preparation of 9-beta-D-arabinofuranosyl-2-fluoroadenine 5'-phosphate (II). In particular, the present invention relates to a process wherein a phosphorylation reaction with 9-beta-D-arabinofuranosyl-2-fluoroadenine (I) is conducted under essentially anhydrous conditions to produce the 5 '-phosphate.

The preparation and use of 9-beta-D-arabinofuranosyl-2-fluoroadenine, known as 2-F-ara-A (NSC 118218), for the treatment of leukemia and as an antiviral agent is well known and is described in U.S. Patent No. 4,188,378 to Montgomery. The problem is that 2-F-ara-A is very cytotoxic. In an effort to reduce the cytotoxicity 9-beta-D-arabinofuranosyl-2-fluoroadenine 5'-phosphate (NSC 312887; 2-F-ara-AMP) was produced. The conventional phosphorylation reactions produced 2-F-araA-AMP in very poor yield and purity. The inability to produce 2-F-araA-AMP in good yields and acceptable purity increased the cost of the drug.

Kim et al J. Carbohydrates Nucleosides-Nucleotides 6(3) 229-236 (1979), Yoshikawa et al. Tet. Letters 50, 5065-5068 (1967), Bull. of the Chem. Soc. Japan 42, 3505-3508 (1969) and Sowa et al., Bull. of The Chem. Soc. Japan 48, 2084-2090 (1975) describe the formation of 5'phosphates of various nucleosides. In the first reference it is suggested that the reaction be conducted in conjunction with the addition of a small amount of water to minimize the formation of diesters. Applicant has found this finding to be unnecessary for the preparation of 2-F-ara-AMP. In fact the Applicant has observed that the reaction proceeds best if water is not added to the reaction mixture.

US-A-4123609 discloses a process for the production of 9-(β-D-arabinofuranosyl)adenine-5'-phosphate by phosphorylating anhydrous 9-(β-D-arabinofuranosyl)adenine with a phosphorous oxyhalide in the presence of a trialkyl phosphate solvent. After the reaction with the phosphorous oxyhalide has been completed, the reaction mixture is hydrolysed with water. It is then necessary to adjust the pH of the resulting aqueous mixture by addition of a base, such as a sodium hydroxide solution, in a sufficient amount to cause separation into an aqueous liquid phase and a non-aqueous liquid phase. The resulting mixture which forms the two liquid phases is subsequently reacted with an inert water-immiscible solvent to remove the trialkyl phosphate therefrom.

It is therefore an object of the present invention to provide an improved process for the preparation of 2-F-ara-A-AMP from 2-F-ara-A in good yield and high purity. These and other objects will become increasingly apparent by reference to the following description.

The present invention relates to a process for preparing 9-beta-D-arabinofuranosyl-2-fluoroadenine 5'-phosphate (II) which comprises:
(a) vacuum drying 9-β-D-arabinofuranosyl-2-fluoroadenine (I) to provide anhydrous (I);
(b) reacting under anhydrous conditions in a reaction mixture the vacuum dried (I) with a molar excess of an anhydrous tri-lower alkyl phosphate and a phosphorous oxyhalide, wherein the halide is selected from bromine and chlorine, at a reduced temperature to produce an intermediate of (II) in the reaction mixture;
(c) adding water to the reaction mixture to hydrolyzse the intermediate and terminate the reaction and provide (II) dissolved in the water;
(d) treating the reaction mixture with a non-polar organic solvent which is a non-solvent for (II) to precipitate (II) from the reaction mixture;
(e) separating the precipitated (II) from the reaction mixture; and
(f) dissolving (II) in water and recrystallizing (II) from water.

It has been found that the 2-F-ara-A must be dried extensively under vacuum and that the reaction must be conducted under essentially anhydrous conditions. The 2-F-ara-A is essentially anhydrous, if not completely anhydrous, as a result of heating under a vacuum to eliminate most of the water of hydration. The tri-lower alkyl phosphate is also dried.

The lower alkyl phosphate is preferably trimethyl phosphate. Tri-lower alkyl phosphates where the alkyl groups are 1 to 2 carbon atoms can be used.

After the reaction to form 2-F-ara-AMP (II) is completed, water is added to hydrolyze the intermediate (a 5'-phosphorodichloridate) and to terminate the reaction. The 2-F-ara-AMP (II) dissolves in the water and then is separated from the water. Preferably a non-polar organic solvent which is essentially a non-solvent for 2-F-ara-AMP is used to cause 2-F-ara-AMP (II) to precipitate. The solvent is removed and preferably the 2-F-ara-AMP (II) is dissolved in water and recrystallized from the water. Small amounts of 2-F-ara-AMP (II) are recovered from the solvent.

### 9-beta-D-Arabinofuranosyl-2-fluoroadenine 5'-phosphate (II) NSC 312887

In exploratory work the nucleoside (I), after air-drying, was dried under vacuum at room temperature for several days to yield a monohydrate. The monohydrate dissolved readily (^{~}20 min) in the reaction medium as the reaction proceeded, the excess phosphorus oxychloride appeared adequate to destroy the water of hydration and the yields were unacceptably low. In further work, the nucleoside (I) was dried 24 hours at 0.33 mmHg and 90°C to give essentially anhydrous material. The trimethylphosphate reaction solvent was distilled and the forerun and tail fractions were discarded. With these changes the time to obtain a homogeneous reaction system was extended to 4 to 6 hours and the yields were both reproducible and markedly improved (75-79%, average 76%) based on product (II) as a monohydrate after drying at room temperature for 4 hours at 0.3 mmHg.

A typical 100 g run used to process 785 g of intermediate (I) is described. Phosphorous oxychloride (80.0 g, 49 mL, 523 mmol) was added to cold (0°C, ice-bath) anhydrous, redistilled trimethylphosphate (1 L) and the solution was kept at 0°C for 1 hour. Dried 9-beta-D-Arabinofuranosyl-2-fluoroadenine (I) (100.0 g, 350.6 mmol) was added with stirring in one portion. The reaction mixture became homogeneous (light-yellow solution) after 4 to 6 hours. The reaction mixture was then placed in a refrigerator (^{~}1°C) for 15 hours. No starting material was present by tlc. Water (70 mL) was added and the solution was stirred for 3 hours at 0°C. The mixture was then poured into cold (-0°C, ice-bath) methylene chloride (8 L) with stirring and held in the ice-bath with stirring until a clear methylene chloride phase was obtained (1 h) The methylene chloride was removed by decantation and the residual yellowish, gummy mass was dissolved in warm (^{~}50°C) water (700 mL). The solution was seeded and allowed to stand at room temperature overnight. The resulting crystalline product was collected by filtration and washed with water (50 mL) and with ethanol (2 x 50 mL). The product (II) was dried at room temperature at 0.3 mmHg for 4 hours to give 78.5 g (tlc, trace impurities) of first crop material, mp 200-205°C (dec), with prior browning at ^{~}185°C.

The methylene chloride supernatant liquid, which remained after the isolation of the crude gummy product, was extracted with water (3 x 500 mL) The water extracts were combined and percolated into a column containing Dowex-50 (acid form) resin (560 x 80 mm). The column was eluted with water and the fractions containing product (by UV monitor and tlc) were combined. The aqueous solution was then concentrated (aspirator) to a smaller volume (ca. 250 mL) and allowed to cool to ambient temperature overnight. The resulting crystalline solid (II) was removed by filtration, washed with a small portion of water followed by ethanol, and dried as above to give 11.0 g of product (II) with the same purity (by tlc) as that of first crop of (II). In a similar manner the mother liquor from the first crop of (II) was treated as described above to give 10.5 g of product of the same purity (tlc) as the other crops. The combined yield was 100 g (70% calculated as the monohydrate).

In this manner, 785 g of well-dried (24 h, 90°C, 0.3 mmHg), essentially anhydrous starting nucleoside (I) was processed to give 799 g (76%, calculated as a monohydrate) of good quality target compound (II). However, in the initial series of runs, 510 g of nucleoside as the monohydrate was processed to give but 351 g (54%).

### Recrystallization

The above material, 1134 g of (II), from five runs was dissolved in preheated deionized water (82°C, 15 mL/g). The compound dissolved in 3-5,minutes at 73-75°C. The solution was filtered through paper and the filtrate was transferred to a 22 L flask. The solution was stirred and cooled rapidly to 45-50°C to minimize product (II) decomposition. At this point, the product (II) started to crystallize and the mixture was allowed to cool slowly overnight to complete the precipitation. If the temperature is allowed to fall to 32-33°C before precipitation is complete, the product will precipitate as a gel which is undesirable. The solution was then cooled (ice-bath) for two hours. The resulting precipitate of (II) was collected by filtration through filter-cloth. The filter cake of (II) was washed successively with cold deionized water (1.25 L) and ethanol (1.8 L).

The product (II) was dried at room temperature at 0.3 mmHg for 24 hours and weighed 916 g as an 0.8 hydrate at this point. The product (II) was dried further at 55-60°C at 0.3 mmHg for 72 hours to give 881 g (82% recovery) of anhydrous material. The average yield of (II) was 67% from the precursor nucleoside (I). The mother liquor can be reworked and additional pure product (II) (ca 10%) isolated.

In view of the extensive handling in the last step, a final recrystallization of (II) was necessary to remove any inadvertently-introduced water-insoluble impurities. The acidic product is, however, unstable in hot water. Some decomposition occurs during the recrystallization and no real improvement in purity results. With careful handling in the last step, it is possible that the final recrystallization can be avoided.

### Materials

trimethylphosphate
Phosphorous oxychloride (d = 1.645)
Methylene chloride
Dowex 50W-X2, 50-100 mesh
Alcohol, 3A, specially denatured

### PHYSICAL AND ANALYTICAL DATA

### 2-Fluoro-ara-adenosine 5'-phosphate, NSC 312887

Melting Point: 202-203°C (dec), browns at 190°C.
Analysis: Calcd for C₁₀H₁₃FN₅O₇P (365.21)

| | Calcd | Found |
|---|---|---|
| C | 32.89 | 32.77 |
| H | 3.59 | 3.74 |
| N | 19.17 | 19.04 |
| F | 5.20 | 4.96 |
| P | 8.48 | 8.40 |

| Ultraviolet Spectral Data: | | | |
|---|---|---|---|
| (0.1 N HCl) | lambda max (H₂O) | 262 nm | 13,500 |
| (0.1 N NaOH) | lambda max (H₂O) | 261 nm | 15,600 |

Thin Layer Chromatograph: (EM Silica gel 60F-254, 240)
iso-PrOH-H₂O-NH₄OH (7:2:1), R_{f} = 0.24, trace impurity,
n-PrOH-MeOH-H₂O-NH₄OH (4:3:2:1), R_{f} = 0.41, trace impurity
MeOH-H₂O-NH₄OH (75:25:1), R_{f} = 0.70, trace impurity

Solubility Data: 25°C, without heating (Ref 1).
Free Acid:
- Water:: 9 mg/mL (8.7 and 9.3 mg/mL), 2 det'ns, pH^{∼}2
- Ethanol:: Insoluble

Sodium Salt:
- Water:: >100 mg/mL (upper limit not determined)

It is intended that the foregoing description be only illustrative of the present invention and that the present invention be limited only by the hereinafter appended claims.

## Claims

1. A process for preparing
9-β-D-arabinofuranosyl-2-fluoroadenine 5'-phosphate (II) which comprises:
(a) vacuum drying
9-β-D-arabinofuranosyl-2-fluoroadenine (I) to provide anhydrous (I);
(b) reacting under anhydrous conditions in a reaction mixture the vacuum dried (I) with a molar excess of an anhydrous tri-lower alkyl phosphate and a phosphorous oxyhalide, wherein the halide is selected from bromine and chlorine, at a reduced temperature to produce an intermediate of (II) in the reaction mixture;
(c) adding water to the reaction mixture to hydrolyzse the intermediate and terminate the reaction and provide (II) dissolved in the water;
(d) treating the reaction mixture with a non-polar organic solvent which is a non-solvent for (II) to precipitate (II) from the reaction mixture;
(e) separating the precipitated (II) from the reaction mixture; and
(f) dissolving (II) in water and recrystallizing (II) from water.

2. The process of claim 1, wherein the tri-lower alkyl phosphate is trimethyl phosphate.

3. The process of claim 1, wherein phosphorous oxyhalide is phosphorous oxychloride.

4. The process of claim 1, wherein the temperature is -2°C to 0°C.

5. The process of claim 1, wherein the reaction in step (b) is conducted for at least 16 to 18 hours after the reaction mixture becomes homogeneous as a light yellow solution.

6. The process of claim 1, wherein the non-polar organic solvent used in step (d) is methylene chloride.

7. The process of claim 1, wherein step (a) is conducted by heating (I) to a temperature between 85° and 90°C for at least 24 hours in a vacuum of less than 26.66 Pa (0.2 mm Hg).

8. The process of claim 6, wherein the methylene chloride supernatant liquid obtained in step (d) is extracted with water to provide (II) in the water, the water with (II) is eluted through an ionic chromatographic column to provide aqueous fractions containing (II); the aqueous fractions are concentrated using a vacuum and heating to remove some of the water; a mixture of the concentrated aqueous fractions containing (II) is cooled to crystallize (II) from the concentrated solution and (II) is separated from the water.

9. The process of claim 1, wherein the separated (II) in step (e) is dissolved in heated water and then the heated water is cooled to precipitate (II) which is separated from the water.

## Patentansprüche

1. Verfahren zum Herstellen von
9-β-D-Arabinofuranosyl-2-fluoradenin-5'-phosphat (II), umfassend:
(a) Vakuumtrocknen von
9-β-D-Arabinofuranosyl-2-fluoradenin (I), um wasserfreies (I) bereitzustellen;
(b) Umsetzen des vakuumgetrockneten (I) unter wasserfreien Bedingungen in einem Reaktionsgemisch mit einem molaren Überschuss von einem wasserfreiem Tri-Niedrigalkylphosphat und einem Phosphoroxyhalogenid, wobei das Halogenid aus Brom und Chlor ausgewählt wird, bei einer reduzierten Temperatur, um ein Zwischenprodukt von (II) in dem Reaktionsgemisch herzustellen;
(c) Zugabe von Wasser zu dem Reaktionsgemisch, um das Zwischenprodukt zu hydrolysieren und die Reaktion zu beenden und in dem Wasser gelöstes (II) bereitzustellen;
(d) Behandeln des Reaktionsgemisches mit einem unpolaren organischen Lösungsmittel, das ein Nichtlösungsmittel für (II) ist, um (II) aus dem Reaktionsgemisch auszufallen;
(e) Abtrennen des ausgefällten (II) von dem Reaktionsgemisch; und
(f) Lösen von (II) in Wasser und Umkristallisieren von (II) aus Wasser.

2. Verfahren nach Anspruch 1, wobei das Tri-Niedrigalkylphosphat Trimethylphosphat ist.

3. Verfahren nach Anspruch 1, wobei das Phosphoroxyhalogenid Phosphoroxychlorid ist.

4. Verfahren nach Anspruch 1, wobei die Temperatur -2°C bis 0°C beträgt.

5. Verfahren nach Anspruch 1, wobei die Reaktion in Schritt (b) mindestens 16 bis 18 Stunden lang durchgeführt wird, nachdem das Reaktionsgemisch homogen wird als hellgelbe Lösung.

6. Verfahren nach Anspruch 1, wobei das in Schritt (d) verwendete unpolare organische Lösungsmittel Methylenchlorid ist.

7. Verfahren nach Anspruch 1, wobei Schritt (a) durch Erhitzen von (I) auf eine Temperatur zwischen 85° und 90°C mindestens 24 Stunden lang in einem Vakuum von weniger als 26,66 Pa (0,2 mm Hg) durchgeführt wird.

8. Verfahren nach Anspruch 6, wobei die überstehende Methylenchlorid-Flüssigkeit, die in Schritt (d) erhalten wird, mit Wasser extrahiert wird, um (II) in dem Wasser bereitzustellen, das Wasser mit (II) durch eine Ionenchromatographie-Säule eluiert wird, um wässrige Fraktionen, die (II) enthalten, bereitzustellen; die wässrigen Fraktionen unter Verwendung eines Vakuums und durch Erhitzen konzentriert werden, um einen Teil des Wassers zu entfernen; ein Gemisch der konzentrierten wässrigen Fraktionen, die (II) enthalten, gekühlt wird, um (II) aus der konzentrierten Lösung auszukristallisieren, und (II) von dem Wasser abgetrennt wird.

9. Verfahren nach Anspruch 1, wobei das abgetrennte (II) in Schritt (e) in erhitztem Wasser gelöst wird und dann das erhitzte Wasser gekühlt wird, um (II) auszufallen, das von dem Wasser abgetrennt wird.

## Revendications

1. Procédé pour préparer du 9-β-D-arabinofuranosyl-2-fluoroadénine 5'-phosphate (II), qui comprend les étapes consistant à :
(a) sécher sous vide de la 9-β-D-arabinofuranosyl-2-fluoroadénine (I) pour fournir de la (I) anhydre ;
(b) faire réagir, dans des conditions anhydres dans un mélange réactionnel, la (I) séchée sous vide avec un excès molaire de phosphate de trialkyle inférieur anhydre et d'un oxyhalogénure de phosphore, dans lequel l'halogène est choisi parmi le brome et le chlore, à une température réduite pour fournir un produit intermédiaire de (II) dans le mélange réactionnel ;
(c) ajouter de l'eau au mélange réactionnel pour hydrolyser le produit intermédiaire et achever la réaction et fournir (II) dissous dans l'eau ;
(d) traiter le mélange réactionnel avec un solvant organique non polaire qui est un non-solvant pour (II), pour précipiter (II) du mélange réactionnel ;
(e) séparer le (II) précipité du mélange réactionnel ; et
(f) dissoudre (II) dans de l'eau et recristalliser (II) à partir d'eau.

2. Procédé selon la revendication 1, dans lequel le phosphate de trialkyle inférieur est le phosphate de triméthyle.

3. Procédé selon la revendication 1, dans lequel l'oxyhalogénure de phosphore est l'oxychlorure de phosphore.

4. Procédé selon la revendication 1, dans lequel la température est de -2 °C à 0 °C.

5. Procédé selon la revendication 1, dans lequel la réaction dans l'étape (b) est effectuée pendant au moins 16 à 18 heures après que le mélange réactionnel est devenu homogène sous la forme d'une solution jaune clair.

6. Procédé selon la revendication 1, dans lequel le solvant organique non polaire utilisé dans l'étape (d) est le chlorure de méthylène.

7. Procédé selon la revendication 1, dans lequel l'étape (a) est mise en oeuvre en chauffant (I) à une température entre 85 et 90 °C pendant au moins 24 heures dans un vide de moins de 26,66 Pa (0,2 mm de Hg).

8. Procédé selon la revendication 6, dans lequel le chlorure de méthylène liquide surnageant obtenu dans l'étape (d) est extrait avec de l'eau pour fournir (II) dans l'eau, l'eau avec (II) est éluée à travers une colonne chromatographique ionique pour fournir des fractions aqueuses contenant (II) ; les fractions aqueuses sont concentrées en utilisant un vide et en chauffant pour enlever un peu de l'eau ; un mélange des fractions aqueuses concentrées contenant (II) est refroidi pour cristalliser (II) à partir de la solution concentrée et (II) est séparé de l'eau.

9. Procédé selon la revendication 1, dans lequel le (II) séparé dans l'étape (e) est dissous dans de l'eau chauffée puis l'eau chauffée est refroidie pour précipiter (II), qui est séparé de l'eau.
